# FASCICULE DE BREVET EUROPEEN

(11) **EP 0 512 912 B1**
(45) Date de publication et mention de la délivrance du brevet: **09.08.1995**
(21) Numéro de dépôt: 92401254.5
(22) Date de dépôt: 04.05.1992
(51) Int. Cl.: C07C 15/00, C07C 2/00, B01J 8/06

(54) **Procédé de production d'hydrocarbures aromatiques dans une enceinte chauffée par des moyens de chauffage radiants à flux thermique variable**
Verfahren zur Herstellung von aromatischen Kohlenwasserstoffen in einem, von strahlenden Heizungsmitteln mit variabeler thermischer Leistung geheizten Gefäss
Process for the production of aromatic hydrocarbons in a reaction enclosure heated by radiant heating means with a variable thermal flux

(30) Priorité: 06.05.1991 FR 9105672
(43) Date de publication de la demande: 11.11.1992
(73) Titulaire: INSTITUT FRANCAIS DU PETROLE, 92502 Rueil-Malmaison (FR)
(72) Inventeur: Renard, Pierre, F-78860 Saint Nom La Breteche (FR); Minkkinen, Ari, F-78860 Saint Nom La Breteche (FR)

(56) Documents cités:
- FR-A- 1 549 226
- US-A- 4 973 778
- PATENT ABSTRACTS OF JAPAN vol. 12, no. 482 (C-553)(3329) 15 Décembre 1988 & JP-A-63 197 534 (MITSUBISHI HEAVY IND) 16 Août 1988

## Description

Cette invention concerne un procédé, un dispositif de conversion d'hydrocarbures et son utilisation notamment dans la production d'hydrocarbures aromatiques à partir d'une charge d'hydrocarbures aliphatiques de 2 à 12 atomes de carbone en présence d'une composition de catalyseur cristallin zéolithique. Elle concerne plus particulièrement la synthèse d'un mélange comprenant en majorité du benzène, du toluène et des xylènes, produits qui sont susceptibles notamment d'améliorer l'indice d'octane des essences.

La valorisation de coupes aliphatiques à bas point d'ébullition telles que les LPG justifie l'intérêt que l'on peut porter à la mise en oeuvre de procédés de conversion de ces hydrocarbures qui soient performants, sélectifs et économiques tout en contribuant également à la formation d'hydrogène comme sous-produit.

La réaction de production d'hydrocarbures aromatiques a été décrite, notamment dans les brevets US 3760 024, US 3756 942 et US 3855 980 en présence d'un catalyseur zéolithique cristallin à base de silice et d'alumine de type MFI comme ZSM5, ZSM12 éventuellement avec un métal comme le gallium dans la charpente ou en présence d'un catalyseur zéolithique contenant un métal hors charpente comme cela est décrit dans les brevets FR 2374 283 et US 4175 057.

Les processus élémentaires mis en jeu dans la transformation des hydrocarbures aliphatiques en hydrocarbures aromatiques sont principalement la déshydrogénation des paraffines, l'oligomérisation des hydrocarbures insaturés obtenus et la cyclisation des oligomères. Globalement, la réaction est fortement endothermique, la vitesse de réaction est sensible aux variations de température et ces réactions successives s'accompagnent d'un dépôt de coke au sein du catalyseur et d'une réduction des oxydes métalliques contenus dans le catalyseur, ce qui le désactive rapidement et réduit la durée de cycle.

Un des problèmes à résoudre consiste donc à assurer une uniformité du chauffage de la zône réactionnelle aux environs de 500 à 600°C permettant d'obtenir un profil de température le plus plat possible dans celle-ci, sachant de plus que le catalyseur est sensible à une augmentation de température et qu'il peut être détruit lorsque la température critique est dépassée.

En effet, on a remarqué que la demande de chaleur n'est pas constante au cours de l'avancement de la réaction endothermique de production d'hydrocarbures aromatiques.

Par ailleurs, un autre problème à résoudre est relatif à la régénération du catalyseur qui doit être rapide, et de fréquence variable suivant la température de la réaction directement dépendante de la charge à traiter mais en général cette régénération est effectuée par exemple toutes les 12 heures. Cette régénération doit être suffisamment douce afin de préserver les performances du catalyseur et minimiser son taux de renouvellement.

Par contre lors de la phase de régénération du catalyseur usagé il est préférable, pour conserver le plus longtemps possible l'activité du catalyseur, d'atteindre le plus rapidement la température de combustion du coke et de maintenir un niveau de température sensiblement constant tout le long du tube.

L'art antérieur décrit un procédé de chauffage par brûleurs disposés sur les parois d'une cellule contenant des tubes remplis de catalyseur. Par exemple, le brevet US-A-4 973 778 décrit l'utilisation de brûleurs et l'utilisation des gaz de combustion comme gaz de régénération du catalyseur. Cette solution présente l'inconvénient de nécessiter un grand nombre de brûleurs radiants conventionnels pour répartir et contrôler les flux thermiques le long des tubes. Par ailleurs, on ne peut disposer qu'une seule rangée de tubes par cellule, ce qui entraîne la multiplication des brûleurs et des cellules étant donné le nombre nécessaire de tubes. De plus ces types de brûleurs et de cellules conduisent à une grande inertie thermique ce qui peut entraîner une détérioration des tubes et du catalyseur contenu dans ces tubes en cas d'arrêt accidentel de l'alimentation de la charge.

Enfin, l'art antérieur est illustré par le brevet JP-A-63197534 (Patent abstracts of Japan (vol. 12, No. 482 (c-553) 3329, 15 dec 1988).

L'objet de l'invention est de répondre aux problèmes soulevés ci-dessus, de façon à améliorer les taux de conversion en hydrocarbures aromatiques et la durée de vie du catalyseur.

Un autre objet est l'utilisation de moyens de chauffage radiants appropriés pour la réaction de production d'hydrocarbures aromatiques et la réaction de régénération du catalyseur dans les mêmes tubes.

Plus particulièrement, l'invention concerne un procédé de production d'hydrocarbures aromatiques à partir d'une charge d'hydrocarbures aliphatiques de 2 à 12 atomes de carbone dans au moins une enceinte réactionnelle comportant une pluralité de tubes sensiblement parallèles, disposés en rangées et contenant un lit fixe d'une composition de catalyseur, ledit procédé étant caractérisé en ce qu'on effectue :
a/ une phase réactionnelle de production d'hydrocarbures aromatiques au cours de laquelle on fait circuler ladite charge, éventuellement préchauffée, dans lesdits tubes contenant la composition catalytique dans des conditions appropriées et on recueille un effluent riche en hydrocarbures aromatiques ;
b/ une phase de purge des tubes avec au moins un gaz approprié, après la phase réactionnelle et après une phase de régénération du catalyseur définie ci-dessous et on recueille un effluent de purge ;
c/ et une phase de régénération, dans lesdits tubes de l'enceinte, du catalyseur en lit fixe sur lequel s'est déposé du coke lors de la phase réactionnelle, dans des conditions de régénération appropriées, et on récupère un effluent de régénération.
ledit procédé étant en outre caractérisé en ce qu'on effectue, lors de la phase réactionnelle, le chauffage des tubes par une pluralité de moyens de chauffage radiants appropriés situés entre deux rangées successives, disposés en nappes sensiblement parallèles, indépendantes entre elles, qui sont sensiblement perpendiculaires aux tubes, lesdits moyens de chauffage étant adaptés à chauffer une première partie (côté alimentation) des tubes avec un flux thermique supérieur au flux thermique moyen de l'enceinte réactionnelle, et à chauffer une deuxième partie des tubes, subséquente de la première avec un flux thermique au plus égal au flux thermique moyen de ladite enceinte de telle façon que l'isothermicité du catalyseur soit sensiblement maintenue, on récupère ledit effluent riche en hydrocarbures aromatiques et on évacue éventuellement de ladite enceinte, des fumées de combustion provenant des dits moyens de chauffage.

Selon une caractéristique du procédé, on peut chauffer de 0,01 à 50 %de la longueur des tubes réactionnels, côté alimentation, avec un flux thermique compris entre 101 et 500 % du flux thermique moyen de l'enceinte et la partie restante des tubes avec un flux thermique compris entre 10 et 100 % du flux thermique moyen de ladite enceinte.

On définit le flux thermique moyen de l'enceinte réactionnelle par le rapport de la puissance absorbée par l'ensemble des tubes pour une réaction donnée sur la somme de la surface extérieure des tubes de l'enceinte.

De manière avantageuse, on peut chauffer de 0,01 à 40 % et de préférence de 0,01 à 35 % de la longueur des tubes réactionnels, côté alimentation avec un flux thermique compris entre 120 et 300 % et de préférence 150 à 200 % du flux thermique moyen et la partie restante des tubes avec un flux thermique compris entre 20 et 85 % et de préférence 40 à 75 % du flux thermique moyen.

Dans ces conditions, le profil de température tout le long du tube est sensiblement plat compte tenu d'une demande plus importante en chaleur dans le première partie du tube que dans la partie restante. Dans ce cas, l'utilisation du catalyseur peut être optimisée en relation avec le quasi maintien de son activité le plus longtemps possible.

Les moyens de chauffage utilisés peuvent avantageusement être ceux décrits dans le brevet US 4664 620 et qui comprennent des brûleurs de forme sensiblement cylindrique allongée, à matrice en fibre de céramique qui brûlent, sans flamme, dans les espaces intersticiels entre les fibres un mélange de combustible gazeux et d'air et qui transfèrent la chaleur par radiation. L'utilisation de ces brûleurs à fibre de céramique est donc un des objets de l'invention.

Ils ont par ailleurs l'avantage de dégager lors de la combustion peu de Noₓ, peu de CO et d'hydrocarbures. Leur utilisation est par ailleurs très souple et d'une grande flexibilité, la quantité de chaleur dégagée pouvant être contrôlée. De plus, ils sont d'une très faible inertie thermique, ce qui est appréciable en cas d'un arrêt inopportun d'alimentation de la charge ou d'un dépôt de coke excessif sur le catalyseur pouvant interrompre l'alimentation de la charge.

Selon une autre caractéristique du procédé, on effectue habituellement une phase de purge entre la phase de production d'hydrocarbures aromatiques et la phase de régénération du catalyseur usagé. Pour ce faire, on stoppe l'alimentation de la charge des tubes, on diminue ou on stoppe le chauffage des tubes, par exemple, en interrompant l'alimentation des brûleurs en comburant et en combustibles et on purge au moins une fois les tubes avec un gaz inerte comme l'azote dans des conditions de débit et de température telles que la température du catalyseur reste sensiblement constante. Cette phase de purge pourra être reproduite entre une phase de régénération et une phase réactionnelle. De ce fait, le catalyseur est sous atmosphère de gaz inerte avant d'être mis en contact soit avec de l'oxygène lors de sa phase de régénération, soit avec des hydrocarbures lors de la phase réactionnelle.

Selon une autre caractéristique du procédé, on effectue en général la phase de régénération du catalyseur en injectant du gaz inerte et de l'air, éventuellement préchauffés, de façon à obtenir un gaz contenant de 0,05 à 3 % en volume d'oxygène moléculaire et de préférence de 0,4 à 0,8 % en volume par rapport au gaz total, dans les tubes et on chauffe de 0,01 à 50 % et de préférence de 0,01 à 35 % de la longueur des tubes côté alimentation avec un flux thermique compris entre 5 et 100 % du flux thermique moyen utilisé lors de la phase réactionnelle dans des conditions telles que l'exothermicité de la réaction de combustion du coke est contrôlée.

Bien évidemment, le passage de la phase de régénération à la phase de production d'hydrocarbures s'effectue en général par une phase de purge comme ci-dessus par exemple qui amènera le catalyseur d'une atmosphère oxydante à une atmosphère inerte.

Selon un mode de réalisation préféré de procédé selon l'invention, l'enceinte réactionnelle peut comprendre au moins un module comportant deux ensembles de tubes disposés en rangées sensiblement parallèles. On effectue en général la phase réactionnelle dans l'un de ces ensembles de tubes pendant que l'on effectue la phase de régénération du catalyseur dans l'autre ensemble de tubes. La régénération étant sensiblement terminée, après la période de purge, les tubes fonctionnant auparavant en régénération peuvent fonctionner alors en réacteur de production d'aromatiques. Ce fonctionnement en alternance par un jeu de vannes contrôlées par des moyens appropriés s'avère très souple.

Le niveau sensiblement identique de la température du lit catalytique durant la phase de production d'hydrocarbures, et de celle nécessaire durant la phase de régénération présente au moins deux avantages :
- Le procédé ainsi décrit permet de réduire le temps de passage entre le cycle de réaction et le cycle de régénération puisque la température est sensiblement la même dans les deux cas et puisqu'il est effectué dans les mêmes tubes.
- Par ailleurs ceci permet de réduire les cycles de contraintes thermiques sur les tubes aussi bien que sur le catalyseur.

La réaction s'effectue en général à une pression comprise entre 0,2 et 10 bar (1 bar = 10⁵ Pa)et à une température de 400 à 600°C en fonction de la nature de la charge, la température étant avantageusement de 480 à 550°C pour la coupe LPG et de 450 à 530°C pour la coupe naphta, et sous une pression préférée de 1 à 5 bar absolus. De préférence cette température est de 500 à 540°C pour la coupe LPG et de 480 à 510°C pour la coupe naphta.

Le catalyseur utilisé est généralement une zéolithe cristalline de type MFI comme les zéolithes ZSM, par exemple ZSM5, ZSM8, ZSM11, ZSM12 et ZSM35 décrites dans le brevet US 3970 544.

Ces zéolithes pourront contenir avantageusement au moins un métal.

A titre d'exemple, on peut citer le zinc, le gallium, de préférence le gallium.

Ces métaux peuvent être dans la charpente ou hors charpente.

On peut également utiliser des zéolithes synthétisées en milieu fluorure avec ou sans métal.

Le catalyseur est utilisé de préférence sous forme de lit fixe, ce qui diminue les phénomènes d'attrition.

Les vitesses spatiales préconisées sont habituellement de 0,5 à 5h⁻¹ et préférentiellement de 1,0 à 3,0h⁻¹.

La charge d'hydrocarbures aliphatiques comprend généralement de 2 à 12 atomes de carbone, elle contient avantageusement du LPG ou du naphta, les conditions opératoires pouvant être différentes suivant la nature de la charge. Par exemple, avec une charge comme le LPG, on pourra opérer à une température supérieure à la température à laquelle on opérerait avec une charge comme le naphta. L'unité permet ainsi de manière très rapide et par un contrôle aisé de la température de l'enceinte, d'accepter des charges de composition variable.

De manière générale, les conditions opératoires sont optimisées de façon à convertir au moins 60 % de la charge, particulièrement avec le LPG pour obtenir un taux d'hydrocarbures aromatiques d'au moins 60 % par rapport à la charge initiale transformée. On peut recycler après séparation des effluents la partie de la charge non convertie.

Des taux de conversion plus importants peuvent être obtenus avec des charges plus lourdes comme un naphta, par exemple au moins 95 %.

La régénération est généralement effectuée à une température comprise entre 450° et 650°C et de préférence de 480 à 560°C.

Le procédé selon l'invention peut être mis en oeuvre par un dispositif de conversion d'hydrocarbures comprenant au moins une enceinte (1) contenant au moins un réacteur (2) comportant une pluralité de tubes (3) sensiblement parallèles entre eux et alimentés en parallèle, les tubes étant remplis d'au moins un catalyseur en lit fixe, des moyens (4) d'alimentation en une charge, reliés à une extrêmité des dits tubes et des moyens de récupération (5) d'un effluent, connectés aux dits tubes, les dits tubes étant disposés en rangées sensiblement parallèles entre lesquelles sont disposés une pluralité de moyens (6) de chauffage radiants tels que des brûleurs à gaz à fibre céramique, disposés en nappes sensiblement parallèles, indépendantes entre elles qui sont sensiblement perpendiculaires aux tubes et qui sont adaptées à chauffer les tubes dans des conditions appropriées.

Le dispositif comporte en outre des moyens de régénération (9, 28) du catalyseur usagé comprenant des moyens de purge des tubes, des moyens (9a, 9b) d'alimentation en gaz de régénération et des moyens d'évacuation (10, 32) de l'effluent de régénération adaptés à régénérer le catalyseur usagé dans les dits tubes, ces moyens de régénération étant connectés aux dits tubes et des moyens (20) de changement en alternance adaptés à relier les tubes alternativement aux moyens (4) d'alimentation en charge et aux moyens (5) de récupération de l'effluent puis aux dits moyens de régénération (9, 28) du catalyseur, ledit dispositif comportant de plus des moyens (50) de contrôle et de régulation de la température des tubes connectés aux dites nappes des moyens de chauffage.

Les moyens de chauffage comprennent généralement une alimentation en carburant et en comburant et des moyens d'évacuation des fumées de combustion connectés à une section de convection elle même reliée à une cheminée. La section de convection peut comprendre un échangeur de chaleur adapté à préchauffer la charge, les gaz de purge et de régénération.

Les tubes utilisés dans le cadre du procédé ont généralement une longueur de 2 à 20 m et leur diamètre interne est de 10 mm à 200 mm. Ils sont disposés en rangées sensiblement parallèles et avantageusement verticales, chaque rangée pouvant contenir une ou plusieurs lignes de tubes. La distance entre axes des tubes est comprise entre 1,5 et 6 fois son diamètre interne. Selon un mode préféré de réalisation, ils sont suspendus et reliés à l'alimentation de la charge et à l'évacuation de l'effluent du côté d'une même extrêmité.

Selon une autre caractéristique, le dispositif comprend habituellement des moyens de régénération adaptés à régénérer le catalyseur usagé dans les mêmes tubes où s'est déroulée la réaction de production d'aromatiques. Ces moyens de régénération comportent en général une alimentation en gaz de régénération à l'une des extrêmités du faisceau de tubes et une évacuation de l'effluent de régénération à l'autre extrêmité, cette extrêmité pouvant se trouver du même côté, par exemple avec des tubes suspendus.

Le dispositif peut comporter également des moyens adaptés à relier les tubes réactionnels alternativement aux moyens de régénération puis aux moyens nécessaires à la réalisation de la réaction, en particulier à relier une extrêmité des tubes aux moyens d'alimentation en charge et l'autre extrêmité aux moyens d'évacuation de l'effluent produit, cette extrêmité pouvant se trouver du même côté dans le cas de tubes suspendus.

Selon un mode de réalisation préféré, le dispositif peut comprendre au moins une enceinte avec un premier réacteur contenant une pluralité de tubes réactionnels et un deuxième réacteur comportant une pluralité de tubes de régénération adaptés à régénérer le catalyseur, le premier et le second réacteur communiquant par un passage avec une section de convection reliée à l'extérieur par des moyens appropriés (cheminée), ces tubes de régénération étant reliés à une extrêmité à une alimentation de gaz de régénération et à l'autre extrémité à une évacuation d'un effluent de régénération et comprenant en outre les moyens de changement en alternance adaptés à relier les tubes réactionnels alternativement aux moyens d'alimentation en charge et aux moyens de récupération de l'effluent, puis à l'alimentation en gaz de régénération et à l'évacuation de l'effluent de régénération, ces moyens de changement en alternance étant adaptés à relier en outre les tubes de régénération alternativement à l'alimentation en gaz de régénération et à l'évacuation de l'effluent de régénération, puis aux moyens d'alimentation en charge et aux moyens de récupération de l'effluent d'hydrocarbures aromatiques, les tubes réactionnels opérant en phase dite réactionnelle pendant que les tubes de régénération opèrent en phase dite de régénération dans un premier temps et les tubes réactionnels devenant ensuite des tubes de régénération tandis que les tubes de régénération deviennent des tubes réactionnels dans un deuxième temps.

La technologie de conception très souple grâce à l'aspect modulaire de sa mise en oeuvre peut être adaptée aussi bien aux petites qu'aux grandes capacités, la souplesse et la flexibilité d'utilisation du chauffage dans de courts délais étant un atout supplémentaire.

Selon une autre caractéristique du dispositif, les moyens de contrôle et de régulation peuvent comprendre au moins une sonde de température reliée à la dite première partie des tubes et au moins une autre sonde de température connectée à la seconde partie subséquente des tubes, ces deux sondes délivrant des signaux, des moyens de traitement et de commande adaptés à recevoir les signaux, à les comparer à des valeurs de référence et à transmettre des informations par d'autres signaux susceptibles d'asservir les moyens de chauffage des premières et deuxièmes parties des tubes.

L'invention sera mieux comprise au vu des figures ci-dessous illustrant de manière schématique le procédé et le dispositif selon l'invention, parmi lesquelles:
- la figure 1 montre une coupe longitudinale de l'enceinte réactionnelle contenant une section réactionnelle et une section de régénération séparée par une section de convection,
- la figure 2 représente les moyens de chauffage des tubes ainsi que les moyens de contrôle et de régulation qui les asservissent.

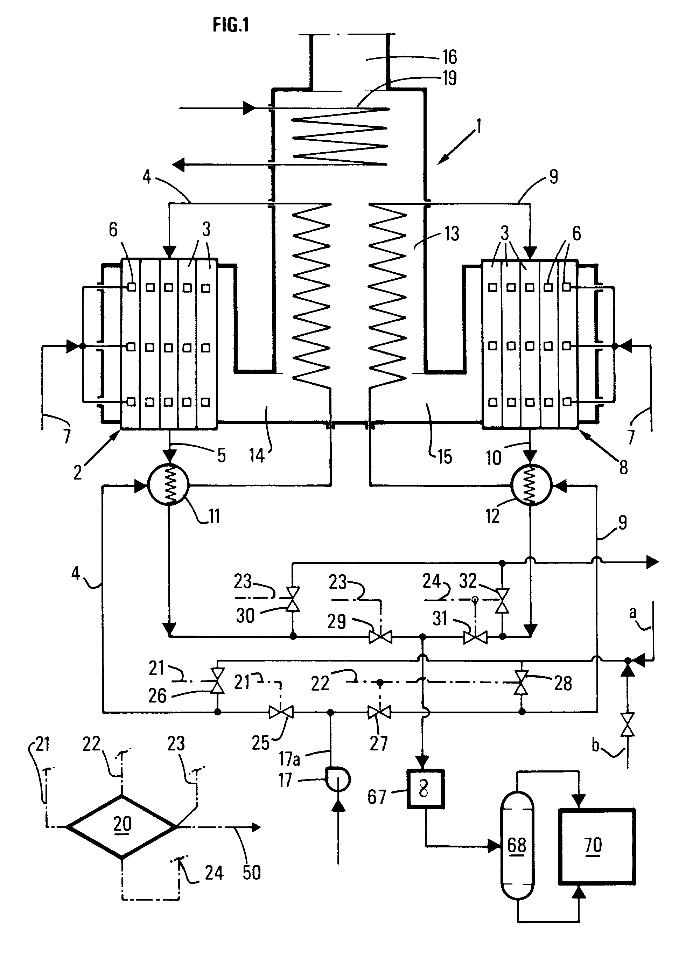

Selon la figure 1, une enceinte réactionnelle 1 ayant des parois revêtues d'un matériau isolant comprend un réacteur 2 comportant une pluralité de tubes 3 en acier inoxydable de forme sensiblement cylindrique qui contiennent un lit fixe d'un catalyseur adapté à la production d'hydrocarbures aromatiques. Ces tubes distants les uns des autres d'une longueur d'environ 2 fois leur diamètre, mesurée à partir de l'axe, sensiblement verticaux sont disposés en rangées sensiblement parallèles, contenant deux lignes de tubes, entre lesquelles sont situés une pluralité de brûleurs 6 de forme sensiblement cylindrique allongée à matrice en fibre de céramique brûlant sans flamme un mélange de combustible gazeux et d'air apporté par une alimentation 7. Ces brûleurs sont disposés en nappes sensiblement parallèles, indépendantes entre elles, qui sont sensiblement perpendiculaires aux tubes réactionnels. Les nappes peuvent comprendre un ou plusieurs brûleurs et la distance entre brûleurs est généralement comprise entre 0,50 et 2,00 m. Par ailleurs la distance entre brûleurs et tubes est généralement comprise entre 0,20 et 0,80 m et avantageusement entre 0,30 et 0,50 m. Ces brûleurs radiants sont adaptés à chauffer une première partie des tubes (côté alimentation) avecun flux thermique d'environ 180% du flux thermique moyen sur une longueur d'environ 30 % de la longueur des tubes qui peut atteindre 8 m. La partie restante des tubes est chauffée avec un flux thermique égal à environ 70 % du flux thermique moyen.

Les fumées de combustion des brûleurs sont évacuées par un passage 14 vers une section de convection 13 dans laquelle la charge peut être chauffée. Un récupérateur de chaleur 19 peut aussi y être incorporé. Les fumées sont évacuées par une cheminée 16.

Les tubes de réacteurs sont alimentés en parallèle par une charge d'hydrocarbures aliphatiques, LPG par exemple, amenée par des lignes 17b et 4, grâce à une pompe 17. Cette charge peut être chauffée par un échangeur thermique 11 en amont de son passage dans la section de convection, cet échangeur recevant de la chaleur de l'effluent du réacteur qui est évacué, en parallèle par une ligne 5 reliée à l'échangeur. L'effluent est ensuite dirigé par un jeu de vannes appropriées 29 vers un aéroréfrigérant 67 puis vers un ballon séparateur 68 de gaz et de liquide et enfin vers une section de fractionnement 70 permettant de recueillir d'une part des hydrocarbures aromatiques et d'autre part un gaz enrichi en hydrogène et un gaz enrichi en hydrocarbures non aromatiques ainsi que la charge non convertie qui peut être recyclée.

L'enceinte réactionnelle comprend selon la figure 1 décrite ci-dessus une cellule de réaction. Elle comprend aussi une cellule de régénération 8 disposée de manière sensiblement symétrique par rapport à l'axe de l'enceinte passant par la section de convection. En fait, cette cellule de régénération est sensiblement identique à la cellule de production d'hydrocarbures puisque les deux réacteurs opèrent de manière alternée, l'une opérant en phase de production d'hydrocarbures pendant que l'autre opère en phase de régénération du catalyseur.

La cellule 8 opérant en régénération comprend donc la pluralité des tubes 3 décrits ci-dessus disposés en rangées entre lesquelles sont situés les brûleurs 6 disposés en nappes et qui sont adaptés à chauffer les tubes dans des conditions de régénération décrites ci-haut. Ces tubes contiennent le catalyseur sur lequel s'est déposé du coke durant la phase précédente de production d'hydrocarbures. Tout d'abord, le gaz utilisé pour la purge (l'azote) amené par la ligne 9a, puis un mélange d'azote et d'air (0,5 % d'oxygène en volume) amené par les lignes 9a et 9b sont introduits après avoir été comprimés à l'entrée du régénérateur 8 par la ligne 9 et la vanne 28. L'alimentation des tubes s'effectue en parallèle. Le catalyseur en lit fixe est au moins partiellement régénéré dans des conditions appropriées et l'effluent de combustion est évacué par une ligne 10 et traverse l'échangeur thermique 12 qui préchauffe partiellement le gaz de purge et le gaz de régénération. Un préchauffage supplémentaire de ces gaz est réalisé également dans la section de convection.

L'effluent de régénération est ensuite dirigé grâce à un jeu de vannes 32 vers une section de traitement et de séparation non représentée sur la figure.

Selon le mode de réalisation déjà décrit, une cellule opère en production d'hydrocarbures pendant que l'autre opère en phase de régénération. Après le temps nécessaire à la réaction de production d'hydrocarbures (12 heures environ), des moyens de changement d'alternance appropriés 20 permettent d'alterner, le réacteur 2 passant en phase de régénération tandis que le régénérateur 8 passe en phase de production d'hydrocarbures.

Pendant la phase nécessaire à la purge, des vannes 26 et 28 alimentant l'ensemble du circuit en azote sont ouvertes, les vannes 25 et 27 étant fermées et des vannes 30 et 32 pour la récupération de l'effluent de purge sont ouvertes pendant que les vannes 29 et 31 sont fermées.

Après la phase de purge, les moyens de changement d'alternance ouvrent la vanne 25 pour l'alimentation du réacteur en la charge et ferment les vannes 26 et 27. Ces mêmes moyens ferment les vannes 30 et 31 pour laisser évacuer l'effluent par la vanne 25 ouverte vers la section de fractionnement 70.

De même, les moyens de changement d'alternance 20 laissent passer le mélange azote et air par la vanne 28 ouverte vers la cellule de régénération 8, la vanne 27 étant fermée tandis que l'effluent de régénération est dirigé vers la section de traitement grâce à la vanne 32 ouverte et la vanne 31 fermée.

Après une nouvelle période de purge, les moyens de changement 20 font passer le réacteur 2 en régénérateur, la ligne 9 du mélange de gaz de régénération alimente la réacteur par la vanne 26 ouverte, les vannes 28 et 29 étant fermées tandis que l'effluent de régénération est évacué par la ligne 5, la vanne 30 étant ouverte, les vannes 29 et 32 étant fermées. De même, les moyens de changement en alternance font passer le régénérateur 8 en réacteur. A cette fin, la charge est envoyée par la ligne 17b et la vanne 27 ouverte, les vannes 25 et 28 étant fermées, vers la ligne 9 alimentant les tubes réactionnels et les effluents de la réaction de production d'hydrocarbures aromatiques sont envoyés vers la section de traitement 70 par la ligne 10, la vanne 31 étant ouverte et les vannes 29 et 32 étant fermées.

Bien entendu, les moyens de changement en alternance 20 sont connectés aux moyens 50 de régulation des nappes de chauffage qui adaptent le chauffage et donc l'alimentation en combustible de manière différente au moment des phases de production d'aromatiques, de purge et de régénération.

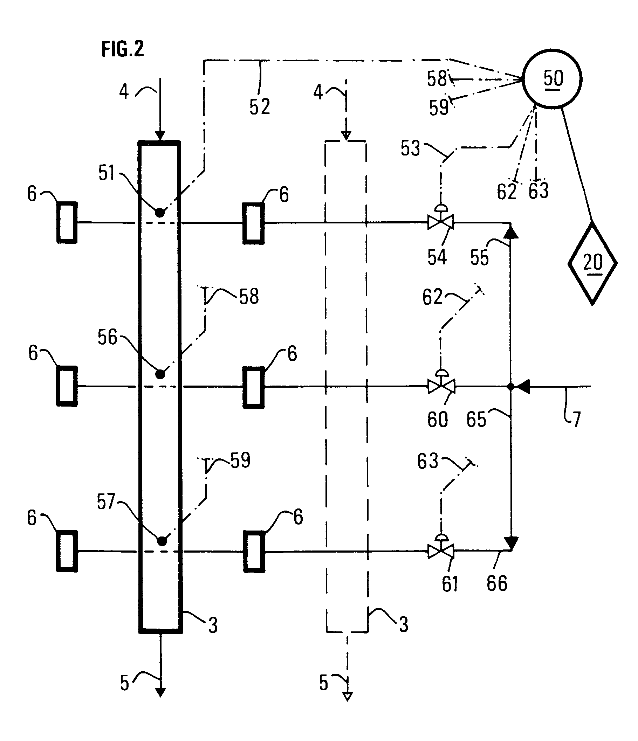

Les moyens de chauffage sont régulés et contrôlés de la manière suivante (figure 2) :
Des sondes de température 51, 56 et 57 disposées sur les tubes réactionnels sensiblement au niveau des différentes nappes de chauffage 6, mesurent la température et envoient un signal électrique respectivement par les lignes électriques 52, 58 et 59 vers les moyens de contrôle et de régulation 50 eux-mêmes connectés aux moyens de changement en alternance 20. Pour une phase déterminée de réaction de production d'aromatiques par exemple, les signaux électriques délivrés par les sondes sont comparés à des valeurs de référence préalablement enregistrées. Les moyens 50 envoient alors des signaux de réponse par les lignes 53, 62 et 63 qui régulent respectivement les vannes d'alimentation en combustible 54, 60 et 61 amené par les lignes 55, 65 et 66 connectées à leur tour à la ligne d'alimentation en combustible 7, ces vannes alimentant en fonction des valeurs de référence et de la température mesurée les moyens de chauffage 6.

On a représenté une enceinte avec un seul module contenant un réacteur ou cellule opérant en phase de production d'hydrocarbures et un autre réacteur opérant en phase de régénération, ces deux réacteurs étant réunis par une section de convection. Bien entendu, cette enceinte peut contenir plusieurs modules, les deux sections de convection adjacents ayant par exemple une seule cheminée d'évacuation des effluents de combustion.

## Revendications

1. Procédé de production d'hydrocarbures aromatiques à partir d'une charge d'hydrocarbures aliphatiques de 2 à 12 atomes de carbone dans au moins une enceinte réactionnelle comportant une pluralité de tubes sensiblement parallèles, disposés en rangées et contenant un lit fixe d'une composition de catalyseur, dans lequel on effectue :
a/ une phase réactionnelle de production d'hydrocarbures aromatiques au cours de laquelle on fait circuler ladite charge, éventuellement préchauffée, dans lesdits tubes contenant la composition catalytique dans des conditions appropriées et on recueille un effluent riche en hydrocarbures aromatiques ;
b/ une phase de purge des tubes avec au moins un gaz approprié, après la phase réactionnelle et après une phase de régénération du catalyseur définie ci-dessous et on recueille un effluent de purge ;
c/ et une phase de régénération, dans lesdits tubes de l'enceinte, du catalyseur en lit fixe sur lequel s'est déposé du coke lors de la phase réactionnelle, dans des conditions de régénération appropriées, et on récupère un effluent de régénération,
ledit procédé étant caractérisé en ce qu'on effectue, lors de la phase réactionnelle, le chauffage des tubes par une pluralité de moyens de chauffage radiants appropriés situés entre deux rangées successives, disposés en nappes sensiblement parallèles, indépendantes entre elles, qui sont sensiblement perpendiculaires aux tubes, lesdits moyens de chauffage étant adaptés à chauffer une première partie représentant de 0,01 à 50% de la longueur des tubes, côté alimentation avec un flux thermique compris entre 101 et 500% du flux thermique moyen de l'enceinte réactionnelle, et à chauffer la partie restante des tubes, subséquentes de la première avec un flux thermique compris entre 10 et 100% du flux thermique moyen de ladite enceinte de telle façon que l'isothermicité du catalyseur soit sensiblement maintenue, on récupère ledit effluent riche en hydrocarbures aromatiques et on évacue éventuellement de la dite enceinte des fumées de combustion provenant des dits moyens de chauffage.

2. Procédé selon la revendication 1 dans lequel on chauffe de 0,01 à 40 % et de préférence 0,01 à 35 % de la longueur des tubes réactionnels côté alimentation avec un flux thermique compris entre 120 et 300% de préférence entre 150 et 200% dudit flux thermique moyen de l'enceinte et la partie restante des tubes réactionnels avec un flux thermique compris entre 20 % et 85 % et de préférence 40 à 75 % du flux thermique moyen.

3. Procédé selon l'une des revendications 1 à 2 dans lequel entre chaque phase réactionnelle et chaque phase de régénération et entre chaque phase de régénération et chaque phase réactionnelle, on stoppe l'alimentation respectivement en la charge et en gaz de régénération, on stoppe éventuellement l'alimentation des brûleurs en carburant et en comburant et on purge au moins une fois les tubes réactionnels avec un gaz inerte tel que l'azote dans des conditions de débit et de température telles que la température du catalyseur reste sensiblement constante.

4. Procédé selon l'une des revendications 1 à 3 dans lequel l'enceinte réactionnelle comporte au moins un module de deux ensembles de tubes, caractérisé en ce qu'on effectue la phase réactionnelle dans un de ces ensembles de tubes pendant que l'on effectue la phase de régénération du catalyseur dans l'autre ensemble de tubes et vice-versa.

5. Procédé selon l'une des revendications 1 à 4 caractérisé en ce que la phase de régénération du catalyseur comporte l'injection d'un gaz inerte tel que l'azote et de l'air, éventuellement préchauffés, de façon à obtenir un gaz contenant de 0,05 à 3 % d'oxygène moléculaire par rapport au gaz total dans les dits tubes réactionnels et le chauffage de 0,01 à 50 % et de préférence de 0,01 à 35 % de la longueur des tubes réactionnels côté alimentation avec un flux thermique compris entre 5 et 100 % du flux thermique moyen de l'enceinte réactionnelle dans des conditions telles que l'exothermicité de la réaction de combustion du coke est contrôlée.

6. Procédé selon l'une des revendications 1 à 5 dans lequel les moyens de chauffage sont des brûleurs à matrice en fibre de céramique.

7. Procédé selon la revendication 6 dans lequel la distance entre brûleurs est de 0,5 à 2 mètres.

8. Procédé selon l'une des revendications 1 à 7, dans lequel on contrôle et on régule l'isothermicité du catalyseur par des moyens de traitement et de commande connectés aux moyens de chauffage radiants et à au moins une première sonde de température reliée à la première partie des tubes et à au moins une seconde sonde reliée à la partie restante des tubes.

## Claims

1. A method of producing aromatic hydrocarbons from a charge of aliphatic hydrocarbons with 2 to 12 carbon atoms in at least one reaction chamber, the chamber having a plurality of substantially parallel tubes arranged in rows and containing a fixed bed of a catalyst composition, wherein:
a) a reaction phase producing aromatic hydrocarbons is carried out, during which the possibly preheated charge is circulated in the tubes containing the catalytic composition under appropriate conditions, and an effluent rich in aromatic hydrocarbons is collected;
b) a phase of purging the tubes with at least one appropriate gas is carried out, after the reaction phase and after a catalyst-regenerating phase defined below, and a purge effluent is collected;
c) and a phase of regenerating the catalyst is carried out in the tubes of the chamber under appropriate regenerating conditions, the catalyst being in a fixed bed and having had coke deposited on it during the reaction phase, and a regeneration effluent is recovered,
characterised in that the tubes are heated during the reaction phase by a plurality of appropriate radiant heating means located between two successive rows and arranged in layers which are substantially parallel, independent of one another and substantially perpendicular to the tubes, the heating means being adapted to heat a first part, representing 0.01 to 50% of the length of the tubes, at the feed side, with a heat flow equal to 101 to 500% of the mean heat flow of the reaction chamber, and adapted to heat the remaining part of the tubes, subsequent to the first part, with a heat flow equal to 10 to 100% of the mean heat flow of the chamber, so that the isothermicity of the catalyst is substantially maintained, the effluent rich in aromatic hydrocarbons is recovered and any combustion fumes emanating from the heating means are possibly discharged from the chamber.

2. The method of claim 1, wherein from 0.01 to 40% and preferably 0.01 to 35% of the length of the reaction tubes at the feed side is heated with a heat flow from 120 to 300% and preferably 150 to 200% of the mean heat flow of the chamber, and the remaining part of the reaction tubes with a heat flow from 20 to 85% and preferably 40 to 75% of the mean heat flow.

3. The method of claim 1 or 2, wherein the charge feed and the regenerating gas feed are respectively stopped between each reaction phase and each regeneration phase and between each regeneration phase and each reaction phase, and wherein the fuel and oxidant feed to the burners may be stopped, and the reaction tubes are purged at least once with an inert gas such as nitrogen, under flow and temperature conditions such that the temperature of the catalyst is substantially constant.

4. The method of any of claims 1 to 3, wherein the reaction chamber comprises at least one module of two sets of tubes, characterised in that the reaction phase is carried out in one of these sets of tubes while the catalyst-regenerating phase is carried out in the other set of tubes and vice versa.

5. The method of any of claims 1 to 4, characterised in that the catalyst-regenerating phase comprises injecting an inert gas such as nitrogen and air, both possibly preheated, so as to obtain a gas containing from 0.05 to 3% of molecular oxygen relative to the total gas in said reaction tubes, and heating from 0.01 to 50% and preferably 0.01 to 35% of the length of the reaction tubes at the feed side with a heat flow equal to 5 to 100% of the mean heat flow of the chamber, under conditions such that the exothermicity of the coke combustion reaction is controlled.

6. The method of any of claims 1 to 5, wherein the heating means are burners with a ceramic fibre matrix.

7. The method of claim 6, wherein the distance between burners is 0.5 to 2 metres.

8. The method of any of claims 1 to 7, wherein the isothermicity of the catalyst is checked and regulated by processing and control means connected to the radiant heating means, to at least one first temperature probe connected to the first part of the tubes and to at least one second probe connected to the remaining part of the tubes.

## Patentansprüche

1. Verfahren zur Herstellung von Kohlenwasserstoffen ausgehend von einem Einsatzstoff aus aliphatischen Kohlenwasserstoffen mit zwei bis 12 Kohlenstoffatomen in mindestens einem Reaktionsgefäß, das eine Vielzahl annähernd paralleler in einer Reihe angeordneten Rohre umfaßt, die ein Festbett mit einer Katalysatorzusammensetzung enthaften, wobei folgendes durchgeführt wird:
a) eine Reaktionsphase zur Herstellung aromatischer Kohlenwasserstoffe, im Laufe derer man den besagten, eventuell vorgewärmten Einsatzstoff in den besagten, die Katalysatorzusammensetzung enthaltenden Rohren unter geeigneten Bedingungen zirkulieren läßt und ein an aromatischen Kohlenwasserstoffen reicher Austragsstoff gewonnen wird;
b) eine Phase zum Spülen der Rohre mit mindestens einem geeigneten Gas, nach der Reaktionsphase und nach einer Phase zur Regenerierung des oben beschriebenen Katalysators, wobei ein Austragsstoff des Spülens gewonnen wird;
c) eine Regenerierungsphase des Festbettkatalysators, auf dem sich der Koks im Laufe der Reaktionsphase abgelagert hat, in den besagten Rohren des Behälters und unter geeigneten Regenerierungsbedingungen, wobei ein Austragsstoff des Regenerierens gewonnen wird,
wobei das Verfahren dadurch gekennzeichnet ist, daß während der Reaktionsphase die Beheizung der Rohre durch eine Vielzahl von strahlenden Heizmitteln durchgeführt wird, die sich geeignet zwischen zwei aufeinanderfolgenden Reihen befinden, in etwa parallelen Lagen unabhängig voneinander angeordnet sind, die zu den Rohren etwa senkrecht stehen, wobei die besagten Heizmittel so ausgelegt sind, daß sie einen ersten 0.01 bis 50% der Rohrlänge darstellenden Teil der Rohre, die Versorgungsseite, mit einem Wärmestrom zwischen einschließlich 101 und 500 % des mittleren Wärmestromes des Reaktionsgefäßes erwärmen und den restlichen Teil der Rohre, der auf den ersten folgt, mit einem Wärmestrom erwärmen, der zwischen einschließlich 10 und 100 % des mittleren Wärmestromes des besagten Reaktionsgefäßes ist, so daß die isothermen Bedingungen des Katalysators in etwa aufrecht erhalten werden, der besagte, an aromatischen Kohlenwasserstoffen reiche Austragsstoff gewonnen wird und gegebenenfalls aus dem Reaktionsgefäß die Verbrennungsabgase, die von den besagten Heizmitteln stammen, entfernt werden.

2. Verfahren nach Anspruch 1, bei dem zwischen 0,01 und 40% und vorzugsweise zwischen 0,01 und 35 % der Länge der Reaktionsrohre auf der Versorgungsseite mit einem Wärmestrom von zwischen einschließlich 120 und 300 % und vorzugsweise zwischen 150 und 200 % des besagten mittleren Wärmestromes des Gefäßes und der restliche Teil der Reaktionsrohre mit einem Wärmestrom zwischen einschließlich 20 und 85% und vorzugsweise 40 bis 75 % des mittleren Wärmestromes erwärmt werden.

3. Verfahren nach einem der Ansprüche 1 bis 2, bei dem zwischen jeder Reaktionsphase und jeder Regenerierungsphase und zwischen jeder Regenerierungsphase und jeder Reaktionsphase die Versorgung mit Einsatzstoff oder Regeneriergas, respektive, angehalten wird, gegebenenfalls die Versorgung der Brenner mit Brennstoff und Sauerstoffträger angehalten wird und die Reaktionsrohre mindestens ein Mal mit einem Inertgas wie Stickstoff unter Durchsatz- und Temperaturbedingungen gespült wird, daß die Temperatur des Katalysators ungefähr konstant bleibt.

4. Verfahren nach einem der Ansprüche 1 bis 3, bei dem des Reaktionsgefäß mindestens ein Modul mit zwei Rohrgebilden umfaßt, dadurch gekennzeichnet, daß die Reaktionsphase in einem dieser Rohrgebilde durchgeführt wird, während die Regenerierungsphase des Katalysators in dem anderen Rohrgebilde durchgeführt wird, und umgekehrt.

5. Verfahren nach einem der Ansprüche 1 bis 4, dadurch gekennzeichnet, daß die Regenerierungsphase des Katalysators das Einblasen eines Inertgases wie Stickstoff und Luft, die gegebenenfalls vorgewärmt sind, umfaßt, und zwar so, daß in den besagten Reaktionsrohren ein Gas erhalten wird, das zwischen 0,05 und 3 % molekularen Sauerstoff bezogen auf das gesamte Gas enthält und das Beheizen von 0,01 bis 50 % und vorzugsweise von 0,01 bis 35 % der Länge der Reaktionsrohre auf der Versorgungsseite mit einem Wärmestrom zwischen einschließlich 5 und 100 % des mittleren Wärmestromes des Reaktionsgefäßes unter Bedingungen, unter denen das exotherme Verhalten der Verbrennungsreaktion des Kokses gesteuert wird.

6. Verfahren nach einem der Ansprüche 1 bis 5, bei dem die Heizmittel Brenner aus einer Matrix aus keramischen Fasern sind.

7. Verfahren nach Anspruch 6, bei dem der Abstand zwischen den Brennern zwischen 0,5 und 2 Metern beträgt.

8. Verfahren nach einem der Ansprüche 1 bis 7, bei dem die Isothermie des Katalysators durch Mittel zur Behandlung und zum Steuern gesteuert und geregelt wird, die mit den strahlenden Heizmitteln und mit mindestens einer ersten Temperatursonde verbunden sind, die an den ersten Teil der Rohre angeschlossen ist und mit mindestens einer zweiten Sonde, die an den übrigen Teil der Rohre angeschlossen ist.
